## Europäisches Patentamt

## European Patent Office

## Office européen des brevets

(11) Veröffentlichungsnummer: **0 377 554**
**A1**

# EUROPÄISCHE PATENTANMELDUNG

(21) Anmeldenummer: **90890003.8**

(22) Anmeldetag: **05.01.90**

(51) Int. Cl.5: **A61B 5/022**

(30) Priorität: **05.01.89 AT 22/89**

(43) Veröffentlichungstag der Anmeldung:
**11.07.90 Patentblatt 90/28**

(84) Benannte Vertragsstaaten:
**AT BE CH DE DK ES FR GB GR IT LI LU NL SE**

(71) Anmelder: **Schauer, Edmund**
**Hauptstrasse 95**
**A-2492 Zillingdorf(AT)**

(72) Erfinder: **Schauer, Edmund**
**Hauptstrasse 95**
**A-2492 Zillingdorf(AT)**

(74) Vertreter: **Beer, Manfred, Dipl.-Ing. et al**
**Lindengasse 8**
**A-1070 Wien(AT)**

(54) **Verfahren zur Messung des Blutdruckes.**

(57) Beim Verfahren zur Messung des Blutdruckes am menschlichen oder tierischen Körper wird der Blutdruck an wenigstens einer Meßstelle durch einen Meßdrucksensor und ein Referenz-Blutdruck an wenigstens einer Referenz-Meßstelle durch einen Referenz-Meßdrucksensor aufgenommen, wobei die Phasenverschiebung ($\phi$) (Zeitverzögerung) des zwischen der Meßstelle und der Referenz-Meßstelle aufgenommenen Blutdrucksignals gemessen wird. Der Referenz- Drucksensor wird mit einem vorwählbaren, veränderbaren Druck an die Meßstelle gepreßt und der Absolut-Blutdruck sowie dessen Verlauf bei bei jedem Herzschlag wird mittels mathematischer Funktionen wie Reziprozität, Interpolation, Fourier-Analyse, Hilberttransformation, Gewichtungen od.dgl. aus dem Meßwert, dem Referenzmeßwert, dem Druck, mit dem der Referenz-Drucksensor an die Meßstelle gepreßt wird und der Phasenverschiebung ($\phi$) berechnet.

Die vom Meßdrucksensor gemessenen Blutdruckamplitudenwerte für die Diastole und die Systole und die Meßdrucksensorempfindlichkeit können mittels der Phasenverschiebung ($\phi$) und der vom Referenz-Meßdrucksensor aufgenommenen Amplitudenwerte des Blutdruckes kalibriert werden.

Die Vorrichtung zur Durchführung des Verfahrens weist einen an der Meßstelle angeordneten Meß-Drucksensor (1,3,4) und einen Referenz-Drucksensor (6) auf. Zum Andrücken des Referenz-Drucksensors (6) gegen die Referenz-Meßstelle ist eine in ihrer Kraft voreinstellbare und veränderbare, von einer Regelstufe (12) gesteuerte Anpreßvorrichtung (5) vorgesehen.

Fig.1

## Verfahren zur Messung des Blutdruckes

Die Erfindung betrifft ein Verfahren zur Messung des Blutdruckes am menschlichen oder tierischen Körper, wobei der Blutdruck an wenigstens einer Meßstelle durch einen Meßdrucksensor und ein Referenz-Blutdruck an wenigstens einer Referenz- Meßstelle durch einen Referenz-Meßdrucksensor aufgenommen wird, wobei die Phasenverschiebung ($\phi$) (Zeitverzögerung) des zwischen der Meßstelle und der Referenz-Meßstelle aufgenommenen Blutdrucksignals gemessen wird.

Eine bekannte Methode ist das Anlegen einer Druckmanschette an die Extremitäten (Arm oder Bein), bei der die Strömungsgeräusche des Blutes zur Bestimmung des diastolischen und systolischen Blutdruckes verwendet werden. Diese Methode erlaubt keine kontinuierliche Überwachung des Blutdruckes und ist nach den heutigen Hygieneansprüchen nicht mehr vertretbar. Außerdem ist die Druckmanschette meistens nur am Oberarm verwendbar. Durch unsachgemäße Anwendung oder Bewegung des Körperteiles, an dem die Druckmanschette angelegt ist, können sich bedeutende Meßfehler ergeben. Durch den hohen Absperrdruck bei der Bestimmung des systolischen Blutdruckes kann dieser nur kurzzeitig überwacht werden und die Anwendung solcher Blutdruckmeßgeräte ist für alte Menschen problematisch (Schockgefahr durch Blutstau). Die Anwendung dieser Geräte ist überdies bei Tieren sehr problematisch. Da das Aufblasen und Ablassen des Druckes in der Druckmanschette sehr zeitaufwendig ist und überdies eine Anpassung an häufig schwankende Blutdruckwerte nicht erfolgen kann, ist eine kontinuierliche Überwachung des Blutdruckes mit derartigen Geräten sehr schwierig.

In der DE-OS 28 48 198 ist eine Meßvorrichtung mit zwei Sensoren angegeben, welche die Bewegungen des Patienten und somit Verspannungen der Haut ausgleicht. Die Sensoren sind elektrisch gegengeschaltet und nahe aneinander angeordnet. Die Zeitverzögerung der Pulswelle zwischen den Sensoren wird bei dieser Vorrichtung nicht gemessen.

In der EP-A-256 159 sind ein Verfahren und eine Vorrichtung beschrieben, bei denen zwei Pulstöne mit Hilfe von zwei an einer aufblasbaren Manschette angeordneten Signalumformern sowie die zeitliche Verzögerung der Pulstöne einer Pulswelle an den Signalumformern ermittelt werden. Diese zeitliche Verzögerung wird mit der Amplitude des zeitlich späteren Pulstones multipliziert und ein Maß für den diastolischen und/oder den systolischen Druck wird aus der Beziehung zwischen diesem Produkt und dem gleichzeitig in der Manschette gemessenen Druck ermittelt. Um jedoch den Verlauf des Blutdruckes zu bestimmen, ist eine Mehrzahl von Messungen bei abnehmendem Druck in der Manschette erforderlich. Eine Bestimmung des Verlaufes des Blutdruckes jedes einzelnen Pulsschlages ist mit dem Verfahren und mit der Vorrichtung nach der EP-A-256 159 nicht möglich.

Aus der Schrift "Sensor 85, Vol. 1, 1.4-1.4.8 (1985)" und der Zeitschrift "Elektronik 24/25.11.1988", Seiten 63-66 ist eine Methode bekannt, die einen aufdrückbaren Drucksensor verwendet, der bei einer neuen Meßstelle mit einem herkömmlichen Manschetten-Blutdruckmeßgerät geeicht werden muß. Bei dieser Methode wird nur die Amplitude berücksichtigt. Diese Methode ist jedoch für eine breite Anwendung und Langzeit-Blutdruckmessungen sowie eine mobile Blutdrucküberwachung nicht geeignet, da Fehler in der Messung auftreten, wenn z.B. die Kontaktierung an der Haut nachläßt, weil keine unmittelbar am Drucksensor vorgenommene automatische und sich gegebenenfalls periodisch wiederholende Kalibrierung der Messung durchführbar ist und weil Änderungen von Einflüssen an der Meßstelle, wie Hauttemperatur, Drucksensor-Temperaturdrift, Umgebungstemperatur, Luftdruckänderungen,Änderungen des Kontaktierungsdruckes sowie Muskel- und Hautbewegungen und die da mit verbundenen Amplitudenschwankungen an der Meßstelle nicht kompensiert werden können und Referenzmeßstellen fehlen.

Die genannten Meßmethoden sind daher für Langzeitmessungen oder Belastungsmessungen, wie z.B. in der Sportmedizin oder Untersuchungen über die Wirksamkeit von pharmazeutischen Präparaten an Versuchspersonen oder Labortieren oder bei Risikopatienten, wie z.B. herz- und kreislaufkranken Menschen, Babys und Menschen und Tieren mit Herzschrittmachern, bei denen die Absolutmessung des diastolischen sowie des systolischen Blutdruckes sowie des Kurvenverlaufes des Blutdruckes erforderlich ist, nur beschränkt einsetzbar, insbesondere wenn man bedenkt, daß von einem Laien oder einem älteren Menschen keine qualifizierte Kenntnis über das Kalibrieren eines Blutdruckmeßgerätes verlangt werden kann.

Aufgabe der Erfindung ist es, ein Verfahren und eine Vorrichtung zur Messung des Blutdruckes anzugeben, durch welche der ganze Verlauf des Blutdruckes jedes einzelnen Pulsschlages gemessen werden kann, wobei sich ändernde Bedingungen an der bzw. den Meßstellen am menschlichen und tierischen Körper ausgeglichen werden können, das auch für Langzeituntersuchungen geeignet ist und das von Laien und mobil einsetzbar ist.

Erfindungsgemäß wird diese Aufgabe dadurch gelöst, daß der Referenz-Drucksensor mit einem vorwählbaren, veränderbaren Druck an die Meßstelle gepreßt wird und daß der Absolut-Blutdruck sowie dessen Verlauf bei jedem Herzschlag mittels mathematischer Funktionen wie Reziprozität, Interpolation, Fourier-Analyse, Hilberttransformation, Gewichtungen od.dgl. aus dem Meßwert, dem Referenzmeßwert, dem Druck, mit dem der Referenz-Drucksensor an die Meßstelle gepreßt wird und der Phasenverschiebung berechnet wird.

Der Blutdruck wird bei diesem Verfahren an einer sich an einer beliebigen Körperstelle befindlichen Meßstelle gemessen. Da hierbei jedoch die bereits beschriebenen Meßfehler auftreten können, ist eine Referenz-Meßstelle vorgesehen, an der ein Referenz-Drucksensor mit einem vorwählbaren, veränderbaren Druck an die Referenz-Meßstelle gepreßt wird. Liegt dieser Anpreßdruck zwischen den Druckwerten für die Diastole und die Systole, ergibt sich zwischen der Meßstelle und der Referenz-Meßstelle eine Phasenverschiebung des Blutdrucksignals, die gemessen wird. Da der Druck, mit dem der Referenz-Drucksensor gegen die Referenz-Meßstelle gedrückt wird, genau bekannt ist, kann mit Hilfe der Phasenverschiebung und dem gleichzeitig an der Referenz-Meßstelle aufgenommenen Referenz-Meßwert der vom Meßdrucksensor aufgenommene Meßwert mittels mathematischer Funktionen korrigiert werden.

Durch dieses Verfahren ist es daher möglich, auch über längere Zeiträume exakte Messungen des Blutdruckes durchzuführen.

Soll nicht nur eine relative Blutdruckmessung durchgeführt werden, sondern eine Absolut-Blutdruckmessung, so kann gemäß einer weiteren Ausführungsform der Erfindung vorgesehen sein, daß die vom Meßdrucksensor gemessenen Blutdruckamplitudenwerte für die Diastole und die Systole und die Meßdrucksensorempfindlichkeit mittels der Phasenverschiebung und der vom Referenz-Meßdrucksensor aufgenommenen Amplitudenwerte des Blutdruckes kalibriert werden.

Diese Kalibrierung kann gemäß einer Ausführungsform der Erfindung so durchgeführt werden, daß zur Kalibrierung der Blutdruckmessung der Druck, mit dem der Referenzdrucksensor an die Meßstelle gepreßt wird, beginnend bei einem Druck, der geringer als der Druck der Diastole ist, im wesentlichen kontinuierlich bis zu einem Druck, der höher als der Druck der Systsole ist, erhöht wird oder umgekehrt, wobei der Meßwert, der Referenz-Meßwert und die Phasenverschiebung laufend gemessen und als Kalibrationsfaktoren in den Meßzyklus eingebracht werden.

Da nunmehr absolute Bezugswerte für den Blutdruck vorliegen, kann der Verlauf des Blutdruckes in Absolut-Werten angegeben werden.

Gemäß einer weiteren Ausführungsform der Erfindung kann die Messung so durchgeführt werden, daß der Druck, mit dem der Referenz-Drucksensor an die Meßstelle gedrückt wird, laufend automatisch so geändert wird, daß die Phasenverschiebung konstant bleibt, was für bestimmte medizinische Anwendungen vorteilhaft ist.

Um die Messung zu verfeinern, kann noch vorgesehen sein, daß von Elektroden gemessene EKG-Impulse oder EEG-Impulse oder Signale von einem Ultraschall-Doppler-Meßgerät, Mikrowellen-Doppler-Meßgerät, Transkraniellen-Doppler-Meßgerät, Magnetfluß-Meßgerät, Blutfluß-Meßgerät nach dem Prinzip der ionisierenden Strahlung oder Röntgenscanner od. dgl. zur Triggerung der Pulswellen verwendet werden.

Wenn die Messung laufend überwacht werden soll, kann vorgesehen sein, daß die gemessenen Daten in geeichten Maßeinheiten alphanumerisch und/oder graphisch in Real-Zeitfunktionen und/oder imaginären Zeitfunktionen weiterverarbeitet und über eine Anzeigeeinheit angezeigt und/oder akustisch durch eine Sprachausgabe-Einheit ausgegeben werden.

Eine vorteilhafte Ausführungsform einer Vorrichtung zur Durchführung des Verfahrens mit einem an der Meßstelle angeordneten Meß-Drucksensor und einem Referenz-Drucksensor ist dadurch gekennzeichnet, daß zum Andrücken des Referenz-Drucksensors gegen die Referenz-Meßstelle eine in ihrer Kraft voreinstellbare und veränderbare, von einer Regelstufe gesteuerte Anpreßvorrichtung vorgesehen ist.

Eine derartige Vorrichtung ist billig und einfach in Aufbau und Verwendung.

Wenn vorgesehen ist, daß der Referenz-Drucksensor auf die Haut geklebt und/oder durch Vakuum, durch ein gasförmiges oder flüssiges, vorzugsweise beheiztes, in einem abgeschlossenen Raum wirkendes oder aus einer verstellbaren druck- und durchflußüberwachten Düse kommendes Medium, piezomechanisch, mechanisch, elektrisch oder durch Haltevorrichtungen, wie Haltegurte mit Klettverschlüssen, Federklammern, Magnetverschlüssen, regelbare Magnetfelder mit Hilfseinrichtungen und Hilfssensoren oder durch eine Kombination dieser Maßnahmen an die Meßstelle gepreßt wird, dann ist der Referenz-Drucksensor einfach an der Meßstelle zu befestigen und der Druck, mit dem der Referenz-Drucksensor an die Meßstelle gepreßt wird, schnell und exakt regelbar.

In einer vorteilhaften Ausführungsform der Erfindung kann vorgesehen sein, daß die Vorrichtung mehrere Meßdrucksensoren enthält. Diese Meßdrucksensoren können am ganzen Körper verteilt sein, wodurch es möglich ist, die Blutdruckverteilung am gesamten Körper zu messen und daraus ein Druckverteilungsbild zu gewinnen, was eine Diagnose über den Durchblutungszustand, den Kreislaufzustand, die Herzfunktion und die Windkesselfunktion der Aterien (Arterienverkalkung der Blutgefäße) ermöglicht.

Vorteilhafterweise kann auch vorgesehen sein, daß der Meß-Drucksensor aus einer Anzahl von Drucksensoren (Array) aufgebaut ist, deren Signale vorzugsweise einzeln verarbeitet werden, wodurch sich die Messung noch genauer durchführen läßt.

Gemäß einer alternativen Ausführungsform der Erfindung kann das Verfahren zur Messung des Blutdruckes so durchgeführt werden, daß der Blutstrom in Strömungsrichtung des Blutes oberhalb der Referenz-Meßstelle mit einem vor wählbaren, veränderbaren Druck abgesperrt oder definiert verringert wird und daß der Blutdruck sowie dessen Verlauf bei jedem Herzschlag mittels mathematischer Funktionen wie Reziprozität, Interpolation, Fourier-Analyse, Hilberttransformation, Gewichtungen od.dgl. aus dem Meßwert, dem Referenzmeßwert, dem Druck, mit dem der Blutstrom abgesperrt wird und der Phasenverschiebung berechnet wird. Dieses Verfahren bietet den zusätzlichen Vorteil, daß die Referenz-Meßstelle und die Stelle, an der der Blutstrom abgesperrt oder definiert verringert wird, unabhängig voneinander optimal am Körper angeordnet werden können.

Vorteilhafterweise kann bei diesem Verfahren vorgesehen sein, daß der Blutstrom in Strömungsrichtung des Blutes oberhalb der Referenz-Meßstelle mit einem vorwählbaren, veränderbaren Druck, der durch in ihrer Intensität und Frequenz regelbare Stoßwellen und/oder Druckwellen und/oder Schallwellen und/oder Ultraschallwellen und/oder regelbare Magnetfelder mit Hilfseinrichtungen und Hilfssensoren erzeugt wird, abgesperrt oder definiert verringert wird und daß der Blutdruck sowie dessen Verlauf jedes Herzschlages mittels mathematischer Funktionen, wie Reziprozität, Interpolation, Fourier-Analyse, Hilberttransformation, Gewichtungen od. dgl. aus dem Meßwert, dem Referenz-Meßwert, der Phasenverschiebung und dem Druck, mit dem der Blutstrom abgesperrt oder verringert wird, berechnet wird, wobei die dem Druck entsprechenden Werte der Intensität und Frequenz der Stoßwellen und/oder Druckwellen und/oder Schallwellen und/ oder Ultraschallwellen und/oder Magnetfelder zur Berechnung verwendet werden. Dadurch ist es nicht mehr erforderlich, Einrichtungen, mit denen der Blutstrom abgesperrt oder gedrosselt werden soll, direkt mit dieser Körperstelle in Verbindung zu bringen.

Eine erfindungsgemäße Ausführungsform einer Vorrichtung ist dadurch gekennzeichnet, daß ein nach dem Dopplerprin zip funktionierender Meßkopf, vorzugsweise ein Ultraschall-Doppler-Meßkopf mit einem integrierten Sensorblock, vorzugsweise einem Drucksensor, mit dem der Anpreßdruck erfaßt wird, mit dem der Meßkopf händisch oder mechanisch auf die Meßstelle gedrückt wird, vorgesehen ist, wobei der Ultraschall-Doppler-Meßkopf durch das Andrücken auf die Meßstelle den Blutfluß absperrt oder definiert verringert, die Veränderung des Blutflusses mißt und der integrierte Anpreßdruck-Sensor den genauen Anpreßdruck registriert, wobei die Meßwerte der Auswerteeinheit (18) zugeführt werden, und wobei die Bedämpfung des Blutflusses auch durch eine Hilfseinrichtung erfolgen kann. Der Ultraschall-Doppler-Meßkopf, der einen Anpreßdruck-Sensor integriert hat, wird händisch oder automatisch z.B. oberhalb der Radialaterie auf die Haut aufgedrückt. Durch das Andrücken des Doppler-Meßkopfes wird der Blutfluß je nach Anpreßdruck, mehr oder weniger, oder vollständig unterbunden. Dieses wird vom Doppler-Meßkopf registriert. Das Doppler-Meßkopf-Signal sowie das Signal des integrierten Anpreß-Sensors, sowie das Blutdruck-Meß-Sensor-Signal und das Signal des Referenz-Sensors werden verarbeitet, wobei die Amplitudenwerte der erwähnten Meß-Signale, sowie deren Phasenlage berechnet werden. Daraus können mathematisch aus den relativen Blutdruckwerten die Absolut-Blutdruckwerte für die Diastole und Systole gebildet werden. Der Ultraschall-Doppler-Meßkopf kann in Ausführungsformen der Erfindung nach den Unteransprüchen integriert sein.

Für einige Anwendungsfälle kann es von Vorteil sein, statt dem Ultraschall-Doppler-Meßkopf einen Ultraschall-Scanner-Meßkopf oder einen Mikrowellen-Doppler-Meßkopf, einen Transkraniellen-Doppler-Meßkopf, einen Magnetfeld-Meßkopf oder einen Meßkopf, der nach dem Prinzip mit ionisierender Strahlung arbeitet, in das Blutdruck-System einzubinden, wobei die Meßköpfe oder eine Hilfseinrichtung den Blutfluß bedämpfen oder unterbinden können. Die gewonnenen Signale werden dann mit den Daten der anderen Blutdruck-Sensoren zur Blutdruck-Bestimmung verwendet.

Weitere vorteilhafte Ausführungsformen der Erfindung ergeben sich aus den Unteransprüchen und den unter Bezugnahme auf die Zeichnungen beschriebenen Ausführungsbeispielen. Es zeigen

Fig. 1 ein Blockschaltbild einer Ausführungsform der Erfindung mit an einem menschlichen Körper angeordneten Sensoren und Anpreßeinrichtungen,

die Fig. 2 bis 6 mittels der Sensoren gemessene Kurvenverläufe des Blutdruckes in Abhängigkeit vom Absperrdruck,

Fig. 7 eine Ausführungsform einer erfindungsgemäßen Vorrichtung als Handmodell,

Fig. 8 ein stationäres PC-Modell,

Fig. 9 ein Armbandmodell,

die Fig. 10 bis 12 Ausführungsformen von Meßdruckköpfen,

Fig. 13 die Ausführungsform eines Meßdruckkopfes für Finger oder andere Extremitäten,

die Fig. 14 und 15 einen Multisensorkopf für punktuelle Messungen,

Fig. 16 ein Gurtmodell,

Fig. 17 ein Modell zur Einführung in Körperöffnungen, wie z.B. Ohren, in die Speiseröhre, in den Analbereich usw. und

Fig. 18 einen Ultraschall-Meß-Kopf mit Drucksensor .

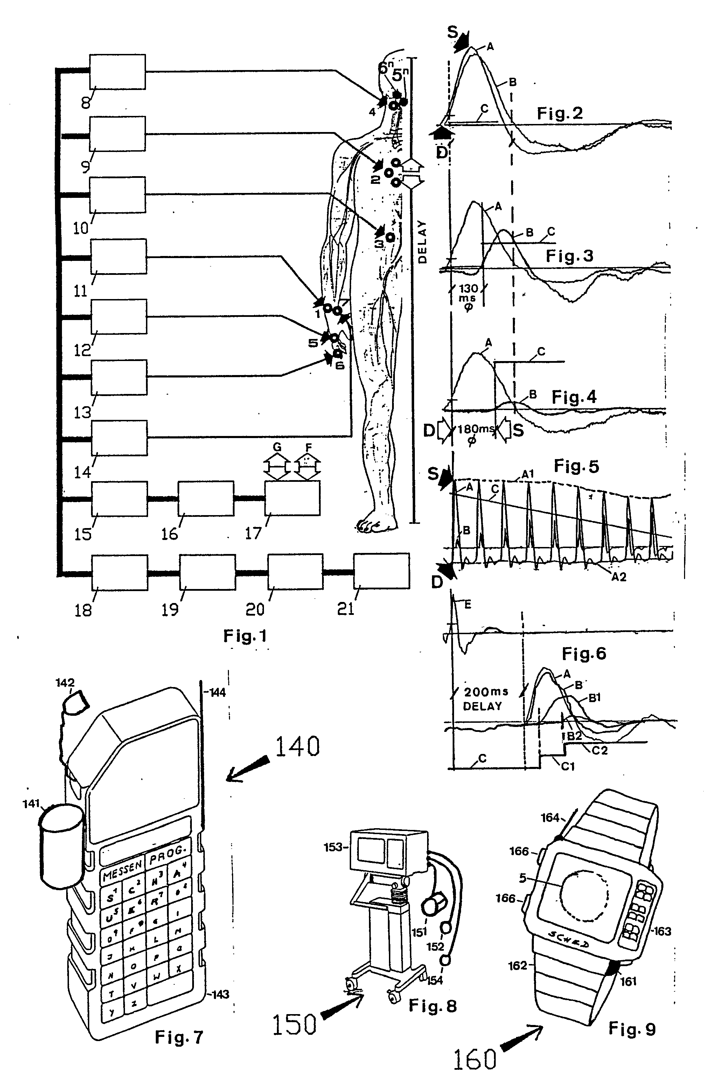

In Fig. 1 ist teilweise ein menschlicher Körper dargestellt, an dem ein Meß-Drucksensor 1 an der Pulsstelle am Handgelenk (A. radial) angeordnet ist, der die Druckänderungen (Dehnungen), die in einem Blutgefäß bei jedem Herzschlag auftreten und an das umgebende Gewebe übertragen werden, an der Hautoberfläche mißt. Da die Blutdrukkamplitude des Meß-Drucksensors 1 von dessen Empfindlichkeit, der Druckvorlast und der Qualität der Kontaktierung an der Hautoberfläche sowie der Beschaffenheit der Meßstelle (wie groß und tief die Blutgefäße unter der Haut liegen) und den Temperaturverhältnissen an und um den Meß-Drucksensor 1 (Haut- und Umgebungsgemperatur) abhängt, ist es erforderlich, dieses vom Meß-Drucksensor 1 erzeugte relative Blutdrucksignal, das bei jedem Herzschlag gemessen wird, mit einem geeichten langzeitstabilen Referenzwert einer ReferenzDruckquelle zu vergleichen, um feststellen zu können, welchem Absolut-Blutdruck das vom MeßDrucksensor 1 gemessene Signal entspricht, um daraus den systolischen und diastolischen AbsolutBlutdruckwert zu gewinnen. Weiters können so die Amplitudenverhältnisse, der Kurvenverlauf, die Anstiegszeit, die Abfallzeit und die Periode des Blutdruckverlaufes genau bestimmt werden.

Als Referenz-Druckquelle ist ein ReferenzDrucksensor 6 vorgesehen. Weiters ist eine Anpreßvorrichtung 5 vorgesehen, durch die der Blutstrom ganz oder auch nur teilweise bzw. überhaupt nicht unterbrochen werden kann. Diese Anpreßvorrichtung 5 kann, wie in Fig. 1 dargestellt, in Strömungsrichtung des Blutes oberhalb des ReferenzDrucksensors 6 angeordnet sein. Es ist jedoch auch möglich, die Anpreßvorrichtung 5 in den Referenz- Drucksensor 6 zu integrieren, wobei die Anpreßvorrichtung 5 den Referenz-Drucksensor 6 gegen die Meßstelle drückt und der Blutstrom auf diese Weise gedrosselt wird.

Am Körper können noch weitere Meß-Drucksensoren 3 und 4 vorgesehen sein, um ein Druckverteilungsbild über den ganzen Körper zu erhalten, wobei dem Meß-Drucksensor 4 eine Anpreßvorrichtung 5n und ein Referenz-Drucksensor 6n zugeordnet sind.

Den Meß-Drucksensoren 1, 3 und 4 sowie dem Referenz-Drucksensor 6 sind jeweils eine nachfolgende Stufe 8, 10, 11 und 13 zugeordnet, die aus einem regelbaren Vorverstärker, einem regelbaren Filter, insbesondere einer Filterkette sowie einem regelbaren Zwischenverstärker bestehen. Der Anpreßvorrichtung 5 ist eine Regelstufe 12 zugeordnet, über die der Anpreßdruck geregelt wird. Den Meß-Drucksensoren 1, 3 und 4 kann auch noch ein Temperatursensor 7 zugeordnet sein, der vorzugsweise in die Meß-Drucksensoren integriert ist, wobei der vom Temperatursensor 7 gemessene Wert über eine Stufe 14 weiterverarbeitet wird.

Werden mehrere Meß-Drucksensoren 1, 3 und · 4 verwendet, so werden die Meßsignale dieser Meß-Drucksensoren 1, 3 und 4 sowie deren eventuell zugeordnete Temperatursignale über einen Multiplexer 15 an eine Auswerteeinheit 18 weitergeleitet. An den Multiplexer 15 oder direkt an die Auswerteeinheit 18 kann eine Alarmstufe 17 über einen Analog-Digital-Wandler 16 angeschlossen sein. Die Alarmstufe 17 kann z.B. ein bidirektionales Interface G, F aufweisen, über das z.B. Computer-Diagnosegeräte, Überlebensmaschinen, Infusionsgeräte, Pumpen oder Fernüberwachungsgeräte, Ultraschall-Doppler-Meßgeräte, Mikrowellen-Doppler-Meß-Geräte, Transkranielle Doppler-Meßgeräte, Magnetfluß-Meßgeräte, Blutfluß-Meßgeräte nach dem Prinzip der ionisierenden Strahlung, Röntgenscanner, od. dgl. angeschlossen sein können.

An die Auswerteeinheit 18 ist eine Speichereinheit 19 sowie eine Anzeigeeinheit 20 und eine Versorgungseinheit 21 angeschlossen.

Wenn, wie in einer Ausführungsform der Erfindung, die Meß-Drucksensoren 1, 3 und 4 sowie der Referenz-Drucksensor 6 aus mehreren Drucksensoren (Array) bestehen, dann kann in die Stufen 8, 10, 1,1 und 13, die vorzugsweise auch in die Drucksensoren integriert sein können, ein Multiplexer eingebaut sein.

Mit 2 sind in Fig. 1 Elektroden bzw. Sensoren bezeichnet, mit denen EKG- oder EEG-Impulse gemessen werden können, mit deren Hilfe die Messung getriggert werden kann. An die Elektroden 2, die vorzugsweise aus leitendem Gummi oder Kunststoff oder einer Metallfolie bestehen, ist eine Stufe 9 angeschlossen, die aus einem regelbaren Vorverstärker, einem regelbaren Filter, insbesondere einer Filterkette sowie einem Zwischenverstärker besteht.

An einem oder mehreren der Meß-Drucksensoren 1, 3, 4 bzw. dem Referenz-Drucksensor 6 kann ein Blutgasanalysator, der Sauerstoffwerte, Kohlendioxidwerte, Alkoholwerte und/oder Acetonwerte od. dgl. mißt, angeordnet sein, der über Vakuum Blut nahe an die Hautoberfläche diffundieren läßt und diese auf die oben angeführten Bestandteile analysiert.

In den Fig. 2 bis 4 sind Kurvenverläufe der von einem Meß-Drucksensor 1 (Kurve A) und dem Referenz-Drucksensor 6 (Kurve B) aufgenommenen Kurvenverläufe des Blutdruckes eines Pulsschlages in Abhängigkeit von der Höhe des Anpreßdruckes (Linie C) dargestellt. Mit D ist der Druck der Diastole bezeichnet und mit S der Druck der Systole.

In Fig. 2 ist der Anpreßdruck C, mit dem der Blutfluß bedämpft wird, kleiner als die Diastole des Blutdruckes und die Phasenverschiebung $\phi$ zwischen der Meßkurve des Meß-Drucksensor 1 und der Meßkurve des Referenz-Drucksensors 6 ist Null (wenn die Sensoren nahe aneinanderliegen, da keine Laufzeitverzögerung auftritt). In Fig. 3 ist der Anpreßdruck C größer als der Druck der Diastole, woraus sich eine im Beispiel von Fig. 3 dargestellte Phasenverschiebung $\phi$ von 130 ms ergibt. Diese Phasenverschiebung $\phi$ (Zeitverzögerung) liegt darin begründet, daß der Anpreßdruck C durch den sich aufbauenden Blutdruck überwunden werden muß, um am nachfolgenden Referenz-Drucksensor 6 ein Signal erzeugen zu können. Dies bewirkt eine zeitliche Verzögerung und damit die Phasenverschiebung $\phi$ größer Null gegenüber dem an einer ungedämpften Meßstelle befindlichen Meß-Drucksensor 1. In den in den Fig. 2 bis 4 dargestellten Messungen wird das vom Meß-Drucksensor 1 gemessene Signal als Trigger verwendet. Es können jedoch auch, wie in Fig. 6 dargestellt, die von den Elektroden 2 angenommenen EKG-Impulse als Trigger verwendet werden oder die Signale eines Ultraschall-Doppler-Meßgerätes.

Wenn nun der Anpreßdruck C (Fig. 4), der von der Anpreßvorrichtung 5 erzeugt wird, größer als der systolische Blutdruck im Blutgefäß ist, erreicht die Phasenverschiebung $\phi$ ihren Maximalwert und die Amplitude des Referenz-Meßdrucksignals wird Null oder annähernd Null, da der Blutdruck den Anpreßdruck (Absperrdruck) nicht überwinden kann. Diese vom Meß-Drucksensor 1 und vom Referenz-Meßdrucksensor 6 gemessenen Werte sowie die gemessene Phasenverschiebung $\phi$ und der Anpreßdruck C der Anpreßvorrichtung 5 werden von der Auswerteeinheit 18 zur Berechnung und Kalibrierung des Meß-Drucksensor 1-Signals für die Blutdruckwerte verwendet und alphanumerisch oder graphisch auf einem Bildschirm der Anzeigeeinheit 20 dargestellt und/oder akustisch durch eine Sprachausgabe-Einheit ausgegeben werden.

In Fig. 5 ist der vollständige Durchlauf des Anpreßdrukkes C der Anpreßvorrichtung 5 von einem Druckwert, der höher als die Systole ist, also das Blutgefäß beinahe vollkommen absperrt (der Amplitudeneinbruch ist zu sehen), bis nahe dem Anpreßdruck C = 0, dargestellt. Es ist zu sehen, wie das Signal A des Meß-Drucksensors 1 in seiner Amplitude schwankt. Die Kurve A1 ist die Einhüllende der Spitzenwerte der Systole S des Meß-Drucksensors 1 und Kurve A2 die Einhüllende der Werte der Diastole D. Das Signal B des Referenz-Drucksensors 6 steigt langsam mit abnehmendem Druck C der Anpreßvorrichtungen, wobei die Phasenverschiebung $\phi$ kleiner wird. Beim Erreichen des diastsolischen Blutdruckes ist keine durch den Anpreßdruck C der Anpreßvorrichtung 5 hervorgerufene Phasenverschiebung $\phi$ mehr zu registrieren.

Dieser Durchlauf der Messung kann verwendet werden, um bei Beginn der Messung das Blutdruckmeßgerät zu kalibrieren, um somit Absolut-Blutdruckwerte zu erhalten. Dieser Durchlauf kann auch während einer Langzeitmessung periodisch in vorbestimmten Zeitabständen laufend wiederholt werden, ohne die Haftung der Sensoren ändern oder überprüfen zu müssen.

Liegen der Meß-Drucksensor 1 und der Referenz-Drucksensor 6 am Körper weit auseinander, so kann durch einen zweiten Meß-Drucksensor 4 in der unmittelbaren Nähe des Referenz-Drucksensors 6 die Pulslaufzeit und die Blutdruckverzögerung in entfernten Körperregionen gemessen und berechnet werden.

Bei einer kontinuierlichen Langzeit-Blutdrucküberwachung kann durch ein ständiges Nachregeln des Anpreßdruckes der Anpreßvorrichtung 5 durch die Steuerstufe 12 eine vordefinierte Phasenverschiebung $\phi$ zwischen dem Meß-Drucksensor 1-Signal und dem Referenz-Drucksensor 6-Signal eingehalten werden. Die Auswerteeinheit 18 berechnet dazu die um die vorgegebene Phasenverschiebung $\phi$ schwankende effektive Phasenverschiebung $\phi$ und regelt den Anpreßdruck der Anpreßvorrichtung 5 über die Steuereinheit 12, um die Phasenverschiebung $\phi$ konstant auf dem eingestellten Wert zu halten.

Wahlweise können zusätzlich zu den normalen Signalwegen über Verzögerungsstufen laufende Signale des Meß-Druck sensors 1 und des Referenz-Drucksensors 6 für eine Drop-out-Kompensation verwendet werden, um beim Ausbleiben von Blutdruckwellen oder Störungen ein dokumentiertes Basis-signal zur Triggerung zur Verfügung zu haben, um das System stabil zu halten, da im Normalfall nicht viele Blutdruckwellen ausbleiben (Herzrythmus-Störungen).

Durch die Alarmstufe 17, die frei wählbare Schwellen (zeitliche Amplitude, Phasenverschiebung, Zeitverzögerung, Frequenzverlauf usw.) besitzt, können gemessene Signale oder Warnsignale akustisch und/oder optisch ausgegeben werden, was über das Interface G, F geschieht, um gegebenenfalls Sicherheitssysteme zu alarmieren oder zu steuern.

In Fig. 6 ist ein von einem Meß-Drucksensor 1 am kleinen Finger und einem Referenz-Drucksensor 6 am Zeigefinger gemessenes Blutdrucksignal dargestellt, wobei die Messung durch EKG-Impulse getriggert wird. Der Kurvenverlauf B, B1 und B2 ist dabei für verschiedene hohe Anpreßdrücke C, C1 und C2 der Anpreßvorrichtung 5 für den Zeigefinger dargestellt. In dieser Darstellung ist die Zeitverzögerung (Delay) zwischen dem Herzimpuls E und der dazugehörigen Pulswelle des Herzen zu erkennen.

In Fig. 7 ist ein Handmodell 140 dargestellt, das einen Multi-Druckmeßkopf 141, einen schwenkbaren und flexiblen Multi-Drucksensor 142 und eine computergesteuerte Einheit 143 mit einer Sende- und Empfangseinheit und einer Antenne 144 aufweist. Im Batteriebetrieb ist diese erfindungsgemäße Ausführungsform des Blutdruckmeßgerätes auch mobil einsetzbar.

In Fig. 8 ist ein PC-Stationärmodell 150 mit einem Multi-Druckmeßkopf 151 und integrierter Absperrvorrichtung, einem Meß-Drucksensor 152, einem Referenz-Drucksensor 154 und einem Personal Computer 153 dargestellt, wobei die Meßdaten auch akustisch durch eine Sprachausgabe-Einheit ausgegeben werden können.

Fig. 9 zeigt ein Armbandmodell 160 mit einem Multi-Drucksensor 161, der vorzugsweise an der Pulsstelle angeordnet wird, ein flexibles und verstellbares Armband 162, eine Computer-Einheit 163, eine Antenne 164 und eine Anpreßvorrichtung 5 sowie externe Sensoranschlüsse 166.

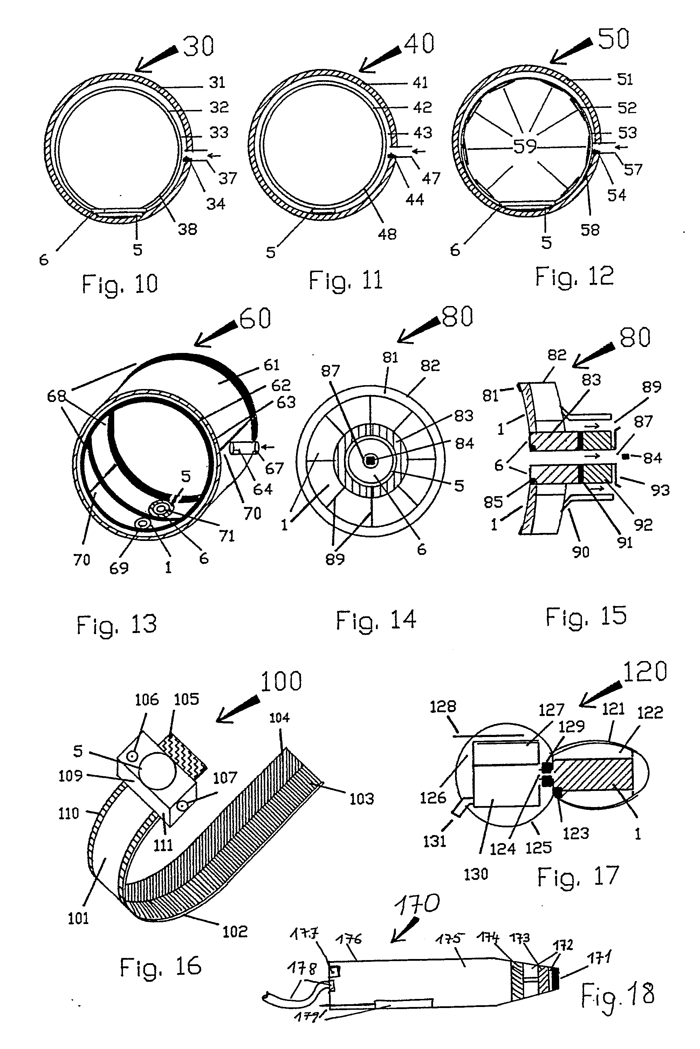

In Fig. 10 ist eine Ausführungsform eines Meß-Druckkopfes 30 dargestellt, der eine stabile Ummantelung 31 aufweist. In dieser Ummantelung 31 ist eine dichte, elastische Hülle 32 angeordnet, die an ihren Rändern mit der Ummantelung 31 verklebt, verschweißt und/oder mit federnden Ringen 38 flüssigkeits- und gasdicht verbunden ist. Zwischen der stabilen Ummantelung 31 und der elastischen Hülle 32 ist ein Zwischenraum 33 für das flüssige oder gasförmige Druckmedium vorgesehen, das durch eine Eintrittsöffnung 37 in den Zwischenraum 33 eingespeist wird, um den Anpreßdruck zu erzeugen. In der Eintrittsöffnung 37 ist ein Anpreß-Drucksensor 34 vorgesehen, über den die effektive Höhe des Anpreßdruckes des Druckmediums ermittelt wird. Zwischen dem Referenz-Drucksensor 6 und der Ummantelung 31 ist ein piezomechanisches Stellglied 5 vorgesehen, das zur schnellen Anpreßdruckregelung für den Referenz-Drucksensor 6 dient.

Die Ausführungsform des Meß-Druckkopfes 40 von Fig. 11 besteht aus einer stabilen Ummantelung 41 und einer elastischen, dichten, druck- und/oder dehnungssensitiven Hülle 42, die vorzugsweise aus einer Piezofolie besteht und als Referenz-Drucksensor verwendet wird. Die Hülle 42 ist an ihren Rändern mit der Ummantelung 41 verklebt, verschweißt und/oder mit federnden Ringen 48 flüssigkeits- und gasdicht verbunden. Zwischen der Hülle 42 und der Ummantelung 41 ist ein Zwischenraum 43 für das Druckmedium gebildet, das durch eine Eintrittsöffnung 47 in den Zwischenraum 43 zugeführt wird und den Anpreßdruck ausübt. Der Anpreßdruck wird über einen Drucksensor 44, der in der Eintrittsöffnung 47 angeordnet ist, gemessen.

Weiters sind piezomechanische Stellglieder 5 zur schnellen Anpreßdruckregelung vorhanden.

In Fig. 12 ist eine weitere Ausführungsform eines Meß-Druckkopfes 50 dargestellt, der aus einer stabilen Ummantelung 51 und einer dichten, elastischen Hülle 52, die an den Rändern mit der Ummantelung 51 verklebt, verschweißt und/oder mit federnden Ringen 58 flüssigkeits- und gasdicht verbunden ist, besteht. Zwischen der Ummantelung 51 und der Hülle 52 ist ein Zwischenraum 53 für das flüssige oder gasförmige Medium gebildet, das durch eine Eintrittsöffnung 57 zugeführt wird, in der ein Anpreß-Drucksensor 54 vorgesehen ist. Der Meß-Druckkopf 50 weist piezomechanische Stellglieder 5 zur schnellen Anpreßdruckregelung, einen Referenz-Drucksensor 6 und eine ringförmige Anordnung von Drucksensoren 59, die insbesondere als Meß-Drucksensoren geschaltet sind, auf. Die Drucksensoren 59 können ebenso als Referenz-Drucksensoren geschaltet sein.

In Fig. 13 ist eine Ausführungsform für einen Meß-Druckkopf 60 dargestellt, die insbesondere für Finger oder derartige Extremitäten geeignet ist. Der Meß-Druckkopf 60 weist eine stabile Ummantelung 61 und eine dichte, elastische Hülle 62, die an den Rändern mit der Ummantelung 61 verklebt, verschweißt und/oder mit federnden Ringen 68 flüssigkeits- und gasdicht verbunden ist, auf. Zwischen der stabilen Ummantelung 61 und der elastischen Hülle 62 ist ein Zwischenraum 63 für das Druckmedium vorgesehen, das durch eine Eintrittsöffnung 67 für das flüssige oder gasförmige Druckmedium, das den Anpreßdruck erzeugt, zugeführt wird. Der Anpreßdruck wird durch einen Anpreß-Drucksensor 64 gemessen. Im ringförmigen Meß-Druckkopf 60 sind ein piezomechanisches Stellglied 5 zur schnellen Anpreßdruckregelung und ein Referenz-Drucksensor 6 vorgesehen, der auf dem piezomechanischen Stellglied 5 angeordnet ist. Weiters ist ein Temperatursensor 71, ein Meß-Drucksensor 1 und ein Blutgasanalysator 69 vorgesehen. Die Ummantelung 61 des Meß-Druckkopfes 60 kann entweder aus zwei Halbschalen, die über gasdichte Scharniere miteinander verbunden sind und beim Schließen dicht miteinander verriegelt werden können oder aus einem einzigen Stück bestehen.

In den Fig. 14 und 15 ist ein überall am Körper verwendbarer Multi-Drucksensor-Analysekopf 80 dargestellt, der einen elastischen Stützkörper 82, der die Sensorsegmente an die Körperform anpaßt und einen Andrucksensor 81 zum Messen des Druckes, mit dem der Kopf 80 an den Körper gepreßt wird, aufweist. Der Kopf 80 besteht weiters aus einem Führungslager 83, das vorzugsweise als Gleitbuchse ausgeführt ist, einem Blutgasanalysataor 84 für eine Blutanalyse, der am Ende eines Vakuumkanals 87 angeordnet ist, und einem Temperatursensor 85. Am Führungslager 83 ist ein Referenz-Drucksensor 6 angeordnet und am elastischen Stützkörper 82 ist eine Anzahl von Meß-Drucksensoren 1 angeordnet, wobei durch die kreisförmige Anordnung der Meß-Drucksensoren 1 die Blutfließrichtung erkannt werden kann. Zwischen dem Führungslager 83 und einer Anpreßvorrichtung 92 ist ein Drucksensor 91 angeordnet. Die Anpreßvorrichtung 92 besteht vorzugsweise aus piezomechanischen Stellgliedern, um den Anpreßdruck rasch steuern zu können. Zwischen einem Befestigungsflansch 90 und dem Führungslager 83 bzw. der Anpreßvorrichtung 92 sind weitere Vakuumkanäle 89 vorgesehen. Am der Meßseite abgekehrten Ende des Analysekopfes 80 ist eine Befestigungsvorrichtung 93 vorgesehen, die so ausgebildet ist, daß der Meßkopf 80 leicht wechselbar ist.

Ein als Meßgurt ausgeführter Meß-Druckkopf 100 ist in Fig. 16 dargestellt. Dieser Meß-Druckkopf 100 besteht aus einem Trägergurt 101, einem elastischen Zwischenteil 102, einer drucksensitiven Fläche 103 als Meß-Drucksensor und einer drucksensitiven Fläche 104 als Referenz-Drucksensor, wobei die Flächen 103 und 104 an der Innenseite des Trägergurtes 101 angeordnet sind. Am Trägergurt 101 ist weiters eine Fixiervorrichtung 105 vorgesehen, um den Gurt schließen zu können. An der Spannvorrichtung 109, die der Fixiervorrichtung 105 zugeordnet ist und über die die Spannung des Trägergurtes 101 elektronisch gesteuert und über Spannzähne 110 durchgeführt werden kann, um den Anpreßdruck zu erzeugen, der zusätzlich von einer Anpreßvorrichtung 5 feingeregelt wird, ist ein Drucksensor 107 mit integriertem Blutanalysator und ein Drucksensor 106 mit integriertem Temperatur-Sensor angeordnet. Weiters ist eine Steuer- und Computer-Einheit 111 integriert, welche die Verarbeitung der Daten durchführt oder gegebenenfalls mit einer externen Verarbeitungs- und Steuereinheit verbunden ist.

Ein in Fig. 17 dargestellter Meß-Druckkopf 120 ist in einen Ohrkanal oder in andere Körperöffnungen einsetzbar und besteht aus einem elastischen, dichten und drucksensitiven Schwellkörper 121, einem Zwischenraum 122 für das Druckmedium und einem Drucksensor 123, der den Anpreßdruck mißt. In einem Gehäuse 125 ist eine Kapillare 124 und ein Druckpuffer 126, eine Pumpe 130 und eine externe Druckmedium-Zuführung 131 für die Pumpe 130 angeordnet. Am Gehäuse 125 sind weiters eine Steuersende- und -empfangsauswerteeinheit 127 und eine Antenne 128 des Regelventils 129 angeordnet. Ein Meß-Drucksensor 1 ist einseitig am Gehäuse 125 befestigt und so an seinen Längsseiten mit dem elastischen Schwellkörper 121 verbunden, daß er nur mit einem geringen Anpreßdruck, der kleiner als die Diastole ist, an die Haut gedrückt wird. Der Schwellkörper 1 ist der Referenz-Drucksensor, der über die Pumpe 130 und die Kapillare 124 mit dem Anpreßdruck beaufschlagt wird, wobei der Meß-Drucksensor 1 so mit dem Schwellkörper verbunden ist, daß sein Anpreßdruck immer kleiner als die Diastole ist.

In Fig. 18 ist ein Multifrequenz-Ultraschall-Doppler-Meßkopf 170 dargestellt. Der Meßkopf 170 arbeitet vorzugsweise gepulst und gegebenenfalls einfach und/oder gewobbelt und/oder moduliert. Der Meßkopf 170 besteht aus ei nem Ultraschall-Sende-Empfangselement 171, das vorzugsweise ein Piezoelement ist, einem Kleber- und Filterteil 172, einen Drucksensor 173 zur Ermittlung der Anpreßkraft, einem Hilfssensorblock 174, einer Steuer- und Auswerteelektronik 175 und einem Gehäuse 176. Am Gehäuse ist ein Anschlußstecker 177 für zusätzliche Blutdruck-Meß-Sensoren, sowie Referenz-Blutdruck-Sensoren und ein Kabel mit Stecker 178 vorgesehen. Der Meßkopf 170 weist eine eigene Stromversorgungseinheit, sowie eine Sende-Empfangseinheit mit einer Antenne 179 für die Übertragung von Meßdaten und Steuerdaten auf, wobei die Sende-Empfangseinheit 179 entweder im Simplex- und/oder Duplex-Sendemodus arbeiten kann und die Daten bei Verwendung mehrerer solcher Vorrichtungen kodiert und/oder in zeitgesteuerter, gepackter Form überträgt, um die Daten gegebenenfalls an ein Hauptgerät zu übertragen. Dadurch kann dieses Gerät auch mobil eingesetzt werden. Falls aus Sicherheitsgründen erforderlich, kann die Datenübertragung und die Energieversorgung über Lichtleiter erfolgen.

Ein Ultraschall-Doppler-Meßkopf kann auch mit den Meß-Drucksensoren 1,3,4 bzw. den Referenz-Drucksensoren 6,6n kombiniert sein, wobei der Ultraschall-Doppler-Meßkopf aus einem Array von Sende-Empfangselementen, vorzugsweise Piezoelementen, Folien, Keramik, Substraten od.dgl. aufgebaut sein kann, um einen möglichst weiten Frequenzbereich überdecken zu können.

Gemäß einer in den Zeichnungen nicht dargestellten Ausführungsvariante der Erfindung kann die Anpreßvorrichtung auch so wirken, daß sie eine gegebenenfalls verstellbare Düse aufweist, durch die ein gasförmiges oder flüssiges Medium ausströmt und dabei den Blutstrom durch direktes Wirken auf die Körperoberfläche absperrt oder definiert verringert, oder daß der aus der Düse austretende Strahl auf den Referenz-Drucksensor wirkt und durch diesen der Blutstrom abgesperrt oder definiert verringert wird.

Die Meß-Drucksensoren 1, 3, 4, der Referenz-Drucksensoren 6,6n und die Anpreßvorrichtung 5 können auch durch andere Haltevorrichtungen, wie Klettverschlüsse mit Federklammern oder Magnetverschlüsse, regelbare Magnetfelder mit Hilfseinrichtungen und Hilfssensoren und/oder selbstklebend, oder durch eine Kombination dieser oder der beschriebenen Maßnahmen an die Körperoberfläche gepreßt werden.

Weiters können auch noch Einrichtungen zur Messung der Atmungsaktivität der Testperson vorgesehen sein, wobei diese Meßwerte ebenfalls zur Berechnung der Auswerteeinheit 18 zugeführt werden können.

Der Referenz-Drucksensor kann in Verbindung mit einer Anpreßvorrichtung z.B. durch ein Ultraschall-Doppler-Meßgerät ersetzt werden, wobei die Anpreßvorrichtung in das Ultraschall-Doppler-Meßgerät integriert oder in Strömungsrichtung des Blutes oberhalb des Ultraschall-Doppler-Meßgerätes angeordnet sein kann.

## Ansprüche

1. Verfahren zur Messung des Blutdruckes am menschlichen oder tierischen Körper, wobei der Blutdruck an wenigstens einer Meßstelle durch einen Meßdrucksensor und ein Referenz-Blutdruck an wenigstens einer Referenz-Meßstelle durch einen Referenz-Meßdrucksensor aufgenommen wird, wobei die Phasenverschiebung ($\phi$) (Zeitverzögerung) des zwischen der Meßstelle und der Referenz-Meßstelle aufgenommenen Blutdrucksignals gemessen wird, dadurch gekennzeichnet, daß der Referenz-Drucksensor mit einem vorwählbaren, veränderbaren Druck an die Meßstelle gepreßt wird und daß der Absolut-Blutdruck sowie dessen Verlauf bei jedem Herzschlag mittels mathematischer Funktionen wie Reziprozität, Interpolation, Fourier-Analyse, Hilberttransformation, Gewichtungen od.dgl. aus dem Meßwert, dem Referenzmeßwert, dem Druck, mit dem der Referenz-Drucksensor an die Meßstelle gepreßt wird und der Phasenverschiebung ($\phi$) berechnet wird.

2. Verfahren zur Messung des Blutdruckes am menschlichen oder tierischen Körper, wobei der Blutdruck an wenigstens einer Meßstelle durch einen Meßdrucksensor und ein Referenz-Blutdruck an wenigstens einer Referenz-Meßstelle durch einen Referenz-Meßdrucksensor aufgenommen wird, wobei die Phasenverschiebung ($\phi$) (Zeitverzögerung) des zwischen der Meßstelle und der Referenz-Meßstelle aufgenommenen Blutdrucksignals gemessen wird, dadurch gekennzeichnet, daß der Blutstrom in Strömungsrichtung des Blutes oberhalb der Referenz-Meßstelle mit einem vorwählbaren, veränderbaren Druck abgesperrt oder definiert verringert wird und daß der Blutdruck sowie dessen Verlauf bei jedem Herzschlag mittels mathematischer Funktionen wie Reziprozität, Interpolation, Fourier-Analyse, Hilberttransformation, Gewichtungen od.dgl. aus dem Meßwert, dem Referenzmeßwert, dem Druck, mit dem der Blutstrom abgesperrt wird und der Phasenverschiebung ($\phi$) berechnet wird.

3. Verfahren nach Anspruch 1 oder 2, dadurch gekennzeichnet, daß die vom Meßdrucksensor gemessenen Blutdruckamplitudenwerte für die Diastole und die Systole und die Meßdrucksensorempfindlichkeit mittels der Phasenverschiebung ($\phi$) und der vom Referenz-Meßdrucksensor aufgenommenen Amplitudenwerte des Blutdruckes kalibriert werden.

4. Verfahren nach einem der Ansprüche 1 oder 3, dadurch gekennzeichnet, daß zur Kalibrierung der Blutdruckmessung der Druck, mit dem der Referenzdrucksensor an die Meßstelle gepreßt wird, beginnend bei einem Druck, der geringer als der Druck der Diastole ist, im wesentlichen kontinuierlich bis zu einem Druck, der höher als der Druck der Systsole ist, erhöht wird oder umgekehrt, wobei der Meßwert, der Referenz-Meßwert und die Phasenverschiebung ($\phi$) laufend gemessen und als Kalibrationsfaktoren in den Meßzyklus eingebracht werden.

5. Verfahren nach einem der Ansprüche 1, 3 oder 4, dadurch gekennzeichnet, daß der Druck, mit dem der Referenz-Drucksensor an die Meßstelle gedrückt wird, automatisch laufend so geändert wird, daß die Phasenverschiebung ($\phi$) konstant bleibt.

6. Verfahren nach Anspruch 2 oder 3, dadurch gekennzeichnet, daß zur Kalibrierung der Blutdruckmessung der Druck, mit dem der Blutfluß oberhalb der Meßstelle des Referenz-Meßdrucksensor abgesperrt oder definiert verringert wird, beginnend bei einem Druck, der geringer als der Blutdruck der Diastole ist, im wesentlichen kontinuierlich bis zu einem Druck, der höher als der Blutdruck der Systole ist, erhöht wird oder umgekehrt, wobei der Meßwert, der Referenz-Meß wert, der Druck und die Phasenverschiebung ($\phi$) laufend gemessen und als Kalibrations-Faktoren in den Meßzyklus eingebunden werden.

7. Verfahren nach einem der Ansprüche 2, 3 oder 6, dadurch gekennzeichnet, daß der Druck, mit dem der Blutstrom abgesperrt oder definiert verringert wird, automatisch laufend so geändert wird, daß die Phasenverschiebung ($\phi$) konstant bleibt.

8. Verfahren nach einem der Ansprüche 2, 3, 6 oder 7, dadurch gekennzeichnet, daß der Blutstrom in Strömungsrichtung des Blutes oberhalb der Referenz-Meßstelle mit einem vorwählbaren, veränderbaren Druck, der durch in ihrer Intensität und Frequenz regelbare Stoßwellen und/oder Druckwellen und/oder Schallwellen und/oder Ultraschallwellen und/oder regelbare Magnetfelder mit Hilfseinrichtungen und Hilfssensoren erzeugt wird, abgesperrt oder definiert verringert wird und daß der Blutdruck sowie dessen Verlauf jedes Herzschlages mittels mathematischer Funktionen, wie Reziprozität, Interpolation, Fourier-Analyse, Hilberttransformation, Gewichtungen od. dgl. aus dem Meßwert, dem Referenz-Meßwert, der Phasenverschiebung ($\phi$) und dem Druck, mit dem der Blutstrom abgesperrt oder verringert wird, berechnet wird, wobei die dem Druck entsprechenden Werte der Intensität und Frequenz der Stoßwellen und/oder Druckwellen und/oder Schallwellen und/oder Ultraschallwellen und/oder Magnetfelder zur Berechnung verwendet werden.

9. Verfahren nach einem der Ansprüche 1 bis 8, dadurch gekennzeichnet, daß von Elektroden gemessene EKG-Impulse oder EEG-Impulse oder Signale von einem Ultraschall-Doppler-Meßgerät, Mikrowellen-Doppler-Meßgerät, Transkraniellen-Doppler-Meßgerät, Magnetfluß-Meßgerät, Blutfluß-Meßgerät nach dem Prinzip der ionisierenden Strahlung oder Röntgenscanner od. dgl. zur Triggerung der Pulswellen verwendet werden.

10. Verfahren nach einem der Ansprüche 1 bis 9, dadurch gekennzeichnet, daß die gemessenen Daten in geeichten Maßeinheiten alphanumerisch und/oder graphisch in Real-Zeitfunktionen und/oder imaginären Zeitfunktionen weiterverarbeitet und über eine Anzeigeeinheit angezeigt und/oder akustisch durch eine Sprachausgabe-Einheit ausgegeben werden.

11. Vorrichtung zur Durchführung des Verfahrens nach einem der Ansprüche 1, 3, 4, 5, 9 oder 10, mit einem an der Meßstelle angeordneten Meß-Drucksensor (1,3,4) und einem Referenz-Drucksensor (6), dadurch gekennzeichnet, daß zum Andrükken des Referenz-Drucksensors (6) gegen die Referenz-Meßstelle eine in ihrer Kraft voreinstellbare und veränderbare, von einer Regelstufe (12) gesteuerte Anpreßvorrichtung (5) vorgesehen ist.

12. Vorrichtung nach Anspruch 11, dadurch gekennzeichnet, daß der Referenz-Drucksensor (6) auf die Haut geklebt und/oder durch Vakuum, durch ein gasförmiges oder flüssiges, vorzugsweise beheiztes, in einem abgeschlossenen Raum wirkendes oder aus einer verstellbaren druck- und durchflußüberwachten Düse kommendes Medium, piezomechanisch, mechanisch, elektrisch oder durch Haltevorrichtungen, wie Haltegurte mit Klettverschlüssen, Federklammern, Magnetverschlüsse, regelbare Magnetfelder mit Hilfseinrichtungen und Hilfssensoren oder durch eine Kombination dieser Maßnahmen an die Meßstelle gepreßt wird.

13. Vorrichtung zur Durchführung des Verfahrens nach einem der Ansprüche 2, 3 oder 6 bis 10, mit einem an der Meßstelle angeordneten Meß-Drucksensor (1,3,4) und einem an der Referenz-Meßstelle angeordneten Referenz-Drucksensor (6), dadurch gekennzeichnet, daß zum Absperren oder definierten Verringern des Blutstromes eine in Strömungsrichtung des Blutes oberhalb des Referenz-Drucksensors (6) angeordnete, in ihrer Kraft voreinstellbare und veränderbare, von einer Regelstufe (12) gesteuerte Anpreßvorrichtung (5) vorgesehen ist.

14. Vorrichtung nach Anspruch 13, dadurch gekennzeichnet, daß der Referenz-Drucksensor (6) auf die Haut geklebt und/oder durch Vakuum, Druckluft, hydraulisch, piezomechanisch, mechanisch, elektrisch oder durch Haltevorrichtungen, wie Haltegurte, Klettverschlüsse, Federklammern, Magnetverschlüsse, regelbare Magnetfelder mit Hilfseinrichtungen und Hilfssensoren, oder durch eine Kombination dieser Maßnahmen an die Meßstelle gepreßt wird.

15. Vorrichtung nach Anspruch 13 oder 14, dadurch gekennzeichnet, daß die Anpreßvorrichtung (5) auf die Haut geklebt und/oder durch Vakuum, Druckluft, hydraulisch, piezomechanisch, mechanisch, elektrisch oder durch Haltevorrichtungen, wie Haltegurte, Klettverschlüsse, Federklammern, Magnetverschlüsse, regelbare Magnetfelder mit Hilfseinrichtungen und Hilfssensoren, oder durch eine Kombination dieser Maßnahmen an die Meßstelle gepreßt wird.

16. Vorrichtung nach einem der Ansprüche 13 bis 15, dadurch gekennzeichnet, daß die Anpreßvorrichtung (5) eine gegebenenfalls verstellbare Düse aufweist, durch die ein gasförmiges oder flüssiges Medium ausströmt und dabei den Blutstrom absperrt oder definiert verringert, wobei der Druck und der Durchfluß überwacht werden können und das ausströmende Medium gegebenenfalls beheizt werden kann.

17. Vorrichtung nach einem der Ansprüche 11 bis 16, dadurch gekennzeichnet, daß der Meß-Drucksensor (1,3,4) auf die Haut geklebt und/oder durch Vakuum, Druckluft, hydraulisch, piezomechanisch oder durch Haltevorrichtungen, wie Haltegurte mit Klettverschlüsse, Federklammern oder Magnetverschlüsse, regelbare Magnetfelder mit Hilfseinrichtungen und Hilfssensoren od.dgl. oder durch eine Kombination dieser Maßnahmen an die Meßstelle gepreßt wird.

18. Vorrichtung nach einem der Ansprüche 11 bis 17, dadurch gekennzeichnet, daß die Vorrichtung mehrere Meß- Drucksensoren (1, 3, 4) enthält.

19. Vorrichtung nach einem der Ansprüche 11 bis 18, dadurch gekennzeichnet, daß jeder Meß-Drucksensor (1, 3, 4) bzw. der Referenz-Drucksensor (6) eine nachfolgende Stufe (8, 10, 11, 13) bestehend aus einem regelbaren Vorverstärker, gefolgt von einem regelbaren Filter, insbesondere einer Filterkette, sowie einen regelbaren Zwischenverstärker, enthält, die an einen von einem Digital-Analog-Konverter gefolgten Multiplexer (15), der die Daten an eine Auswerteeinheit (18) weiterleitet, angeschlossen ist.

20. Vorrichtung nach einem der Ansprüche 11 bis 19, dadurch gekennzeichnet, daß der Meß-Drucksensor (1,3,4) aus einer Anzahl von Drucksensoren (Array) aufgebaut ist, deren Signale vorzugsweise einzeln verarbeitet werden.

21. Vorrichtung nach Anspruch 20, dadurch gekennzeichnet, daß der Meß-Drucksensor (1) eine Anzahl von Drucksensoren (Array) mit integrierter Stufe (11) und einen Multiplexer umfaßt.

22. Vorrichtung nach einem der Ansprüche 11 bis 21, dadurch gekennzeichnet, daß der Referenz-Drucksensor (6) eine Anzahl von Drucksensoren (Array) enthält, deren Signale vorzugsweise einzeln verarbeitet werden.

23. Vorrichtung nach Anspruch 22, dadurch gekennzeichnet, daß der Referenz-Drucksensor (6) eine Anzahl Drucksensoren (Array) mit integrierter Stufe (13) und einen Multiplexer umfaßt.

24. Vorrichtung nach einem der Ansprüche 11 bis 23, dadurch gekennzeichnet, daß der Meß-Drucksensor (3) eine Anzahl von Drucksensoren (Array) mit integrierter Stufe (10) und einen Multiplexer umfaßt.

25. Vorrichtung nach einem der Ansprüche 11 bis 24, dadurch gekennzeichnet, daß die Filter insbesondere die Filterketten der Stufen (8, 9, 10, 11, 13, 14, 18) aus einem Signalprocessor bestehen.

26. Vorrichtung nach einem der Ansprüche 11 bis 25, dadurch gekennzeichnet, daß eine Alarmstufe (17) mit einer Schnittstelle (G, F) vorgesehen ist, welche die Daten eines Laser-Blut-Mikrozirkulationsmeßgerätes (z.B. Laser-Speckle-Methode) in die Blutdruck-Bildauswertung einbezieht.

27. Vorrichtung nach einem der Ansprüche 11 bis 26, dadurch gekennzeichnet, daß über die Schnittstelle (G, F) der Alarmstufe (17) ein Ultraschall-Doppler-Meßgerät oder ein Mikrowellen-Doppler-Meßgerät od.dgl. mit dieser gekoppelt ist, dessen Signale zur Triggerung und Auswertung verwendet werden.

28. Vorrichtung nach einem der Ansprüche 11 bis 27, dadurch gekennzeichnet, daß der Meß-Drucksensor (1) die Stufen (11, 12, 15, 16, 17, 18, 20) sowie eine Speichereinheit (19) und eine Versorgungseinheit (21) aufweist.

29. Vorrichtung nach einem der Ansprüche 11 bis 28, dadurch gekennzeichnet, daß der Referenz-Drucksensor (6) die Stufen (1, 3, 8, 10, 11, 12, 13, 15, 16, 17, 18, 19, 20, 21) aufweist.

30. Vorrichtung nach einem der Ansprüche 11 bis 29, dadurch gekennzeichnet, daß ein im Meß-Drucksensor (1,3,4) integrierter Temperatursensor (7) eine Stufe (14), bestehend aus einen regelbaren Vorverstärker, einem regelbaren Filter, insbesondere einer Filterkette sowie einem regelbaren Zwischenverstärker, enthält.

31. Vorrichtung nach einem der Ansprüche 11 bis 30, dadurch gekennzeichnet, daß am Meß-Drucksensor (1, 3, 4) und/oder am Referenz-Drucksesor (6) ein Sauerstoff, Kohlendioxyd und/oder Alkohol und/oder Azeton messender Blutanalysator, vorzugsweise ein Blutgasanalysator, der über Vakuum Blut nahe an die Hautoberfläche diffundieren läßt und dieses auf die oben angeführten Bestandteile analysiert, vorgesehen ist.

32. Vorrichtung nach einem der Ansprüche 11 . bis 31, dadurch gekennzeichnet, daß der Meß-Drucksensor (1, 3, 4) und der Referenz-Drucksensor (6) Dehnungsmeßstreifen für Dehnung, Druck, Kraft und/oder Hilfssensoren, z.B. für Haftung und Leitfähigkeit, integriert haben.

33. Vorrichtung nach einem der Ansprüche 11, 12 oder 17 bis 32, dadurch gekennzeichnet, daß ein Meßdruckkopf (40) eine stabile Ummantelung (41), eine dichte elastische druck- und/oder dehnungssensitive Hülle (42) vorzugsweise aus einer Piezofolie, die als Referenz-Drucksensor verwendet wird und an den Rändern mit der Ummantelung (41) verklebt, verschweißt und/oder mit federnden Ringen (48) flüssigkeits- und gasdicht befestigt ist, einen Zwischenraum (43) für das Druckmedium, eine Eintrittsöffnung (47) in den Zwischen raum (43) für das Druckmedium, das den Anpreßdruck ausübt, einen Anpreß-Drucksensor (44) und piezomechanische Stellglieder (5) zur schnellen Anpreß-Druckregelung, enthält.

34. Vorrichtung nach einem der Ansprüche 11, 12 oder 17 bis 32, dadurch gekennzeichnet, daß ein Meßdruckkopf (30) eine stabile Ummantelung (31), eine dichte elastische Hülle (32), die an den Rändern mit der Ummantelung (31) verklebt, verschweißt und/oder mit federnden Ringen (38) flüssigkeits- und gasdicht befestigt ist, einen Zwischenraum (33) für das flüssige oder gasförmige Druckmedium, eine Eintrittsöffnung (37) in den Zwischenraum (33) für das Druckmedium, das den Anpreßdruck erzeugt, einen Anpreß-Drucksensor (34), den Referenz-Drucksensor (6) und piezomechanische Stellglieder (5) zur schnellen Anpreß-Druckregelung enthält.

35. Vorrichtung nach einem der Ansprüche 11, 12 oder 17 bis 32, dadurch gekennzeichnet, daß ein Meßdruckkopf (50) eine stabile Ummantelung (51), eine dichte elastische Hülle (52) die an den Rändern mit der Ummantelung (51) verklebt, verschweißt und/oder mit federnden Ringen (58) flüssigkeits- und gasdicht befestigt ist, einen Zwischenraum (53) für das flüssig-oder gasförmige Druckmedium, eine Eintrittsöffnung (57), einen Anpreß-Drucksensor (54), piezomechanische Stellglieder (5) zur schnellen Anpreß-Druckregelung, den Referenz-Drucksensor (6) und eine Anordnung von Drucksensoren (59), die insbesondere als Meß-Drucksensoren und/oder Refrenz-Drucksensoren geschalten sind, enthält.

36. Vorrichtung nach einem der Ansprüche 11, 12 oder 17 bis 32, dadurch gekennzeichnet, daß ein Meßdruckkopf (60) für Finger oder andere Extremitäten eine stabile Ummantelung (61), eine dichte elastische Hülle (62), die an den Rändern mit der Ummantelung (61) verklebt, verschweißt und/oder mit federnden Ringen (70) flüssigkeits- und gasdicht befestigt ist, einen Zwischenraum (63) für das Druckmedium, eine Eintrittsöffnung (67) für das flüssige oder gasförmige Druckmedium, das den Anpreßdruck erzeugt, einen Anpreß-Drucksensor (64), den Referenz-Drucksensor (6), einen Temperatursensor (65), piezomechanische Stellglieder (72) zur schnellen Anpreß-Druckregelung, den Meß-Drucksensor (1), einen Blutgasanalysator (69) und gasdichte Scharniere (71) enthält, wobei die beiden Halbschalen der Ummantelung (61) beim Zuklappen dicht verriegelt werden.

37. Vorrichtung nach Anspruch 36, dadurch gekennzeichnet, daß die Ummantelung (61) aus einem Stück besteht.

38. Vorrichtung nach einem der Ansprüche 11, 12 oder 17 bis 32, dadurch gekennzeichnet, daß ein überall am Körper verwendbarer Multi-Drucksensor-Analyse-Kopf (80) einen Andruck-Sensor (81 ), einem elastischen Stützkörper (82), der die Sensor-Segmente an die Körperform anpaßt, ein Führungslager (83), vorzugsweise eine Gleitbüchse, einen Blutgasanalysator (84), einen Temperatursensor (85), den Referenz-Drucksensor (6), einen Vakuum-Kanal (87) für die Blutanalyse, eine Anzahl von Meß-Drucksensoren (1), eine Anzahl von Vakuum- Kanälen (89), einen Befestigungs-Flansch (90), einen Druck-Sensor (91), eine Anpreßvorrichtung (92), vorzugsweise aus piezomechanischen Stellgliedern bestehend und eine Befestigungsvorrichtung (93) enthält, wobei der Multi-Drucksensor-Analyse-Kopf durch die kreisförmige Anordnung der Meß-Drucksensoren (1) die Blutfließrichtung erkennt und der Meß-Kopf (80) wechselbar ist.

39. Vorrichtung nach einem der Ansprüche 11, 12 oder 17 bis 32, dadurch gekennzeichnet, daß ein als Meßgurt ausgeführter Meßdruckkopf (100) aus einem Träger-Gurt (101 ), einem elastischen Zwischenteil (102), einer drucksensitiven Fläche (103) als Meß-Drucksensor, einer drucksensitiven Fläche (104) als Referenz-Drucksensor, einer Fixiervorrichtung (105), einem Drucksensor (106) mit integriertem Temperatursensor, einem Drucksensor (107) mit integriertem Blutgasanalysator, einer vorzugsweise aus piezomechanischen Stellgliedern bestehenden Anpreßvorrichtung (5), einer Spannvorrichtung (109), die die Spannung des Trage-Gurtes (101) elektronisch gesteuert z.B. über Spannzähne (110) durchführt, um den Anpreß-Druck zu erzeugen, der dann von der Anpreßvorrichtung (5) feingeregelt wird und aus einer Steuer- und &omputereinheit (111) besteht, die die Verarbeitung der Daten durchführt oder gegebenenfalls mit einer externen Verarbeitungs-und Steuereinheit verbunden ist.

40. Vorrichtung nach einem der Ansprüche 11, 12 oder 17 bis 32, dadurch gekennzeichnet, daß ein Meßdruckkopf (120), der in einem Ohrkanal oder in andere Körperöffnungen einsetzbar ist, aus einem elastischen, dichten und drucksensitiven Schwellkörper (121), einem Zwischenraum (122) für das Druckmedium, einem Drucksensor (123) für den Anpreßdruck, einer Kapilare (124) eines Gehäuses (125), einem Druckpuffer (126), einer Steuer-Sende-Empfangs-Auswerteeinheit (127), einer Antenne (128) eines Regelventils (129) für die Anpreßdruckregulierung, einer Pumpe (130), einer externen Druckmediumzuführung (131) und Anschluß für die Kommunikationsleitung, und dem Meß-Drucksensor (1), der einseitig am Gehäuse (125) befestigt und an seinen Längsseiten mit dem elastischen Schwellkörper (121) verbunden ist, besteht, wobei der Schwellkörper (121) den Referenz-Drucksensor bildet.

41. Vorrichtung nach einem der Ansprüche 34 bis 40, dadurch gekennzeichnet, daß die Anpreßvorrichtung (5, 72, 92, 129) mechanisch, elektromechanisch, mit Druckluft, hydraulisch, durch Thermoexpansion wirkend, als Stellmotor, als Magnet oder als regelbare Magnetfelder mit Hilfseinrichtungen und Hilfssensoren ausgebildet ist.

42. Vorrichtung nach einem der Ansprüche 11, 12 oder 17 bis 32, dadurch gekennzeichnet, daß ein Handmodell (140) einen Multi-Druck-Meßkopf (141) mit integrierter Anpreßvorrichtung (Absperrvorrichtung), einen flexiblen Multi- Drucksensor (142) und eine computergesteuerte Einheit (143) mit Sende-Empfangseinheit und Antenne (144) umfaßt.

43. Vorrichtung nach einem der Ansprüche 11, 12 oder 17 bis 32, dadurch gekennzeichnet, daß ein PC-Modell (150) einen Multi-Druck-Meßkopf (151) mit integrierter Anpreßvorrichtung (Absperrvorrichtung) ein Meß-Drucksensor (152), einen Referenz-Drucksensor (154) und einen Personal Computer (153) umfaßt.

44. Vorrichtung nach einem der Ansprüche 11, 12 oder 17 bis 32, dadurch gekennzeichnet, daß ein Armband-Modell (160) einen Multi-Drucksensor (161) für vorzugsweise die Pulsstelle, ein flexibles, verstellbares Armband (162), eine Computereinheit (163), eine Antenne (164), eine Anpreßvorrichtung (5) und externe Sensoranschlüsse (166) umfaßt.

45. Vorrichtung nach einem der Ansprüche 19 bis 44, dadurch gekennzeichnet, daß die Filter und/oder Filterketten aus regelbaren Filtern bestehen, eine Tiefpaß-, Hochpaß-, Bandpaß- oder Kerbfiltercharakteristik aufweisen und vorzugsweise ein Signalprocessor, gegebenenfalls eine Schaltkondensatorfilter, Analogfilter oder Digitalfilter sein können, wobei die Auswerteeinheit (18) einen Signalprocessor und einen von einer Main-CPU gesteuerten Mathematikprocessor bein halten kann und die Anzeigeeinheit (20) aus einer Kathodenstrahlröhre oder einem Flüssigkristalldisplay oder LED-Display oder Luminiszenzdisplay besteht.

46. Vorrichtung nach einem der Ansprüche 11 bis 45, dadurch gekennzeichnet, daß ein nach dem Dopplerprinzip funktionierender Meßkopf, vorzugsweise ein Ultraschall-Doppler-Meßkopf mit einem integrierten Sensorblock, vorzugsweise einem Drucksensor, mit dem der Anpreßdruck erfaßt wird, mit dem der Meßkopf händisch oder mechanisch auf die Meßstelle gedrückt wird, vorgesehen ist, wobei der Ultraschall-Doppler-Meßkopf durch das Andrücken auf die Meßstelle den Blutfluß absperrt oder definiert verringert, die Veränderung des Blutflusses mißt und der integrierte AnpreßdruckSensor den genauen Anpreßdruck registriert, wobei die Meßwerte der Auswerteeinheit (18) zugeführt werden und wobei die Bedämpfung des Blutflusses auch durch eine Hilfseinrichtung erfolgen kann.

47. Vorrichtung nach einem der Ansprüche 11 bis 32, 45 oder 46, gekennzeichnet durch einen Meßkopf (170), vorzugsweise ein gepulster, gegebenenfalls einfach und/oder gewobbelt und/oder moduliert arbeitender Multifrequenz-Ultraschall-Doppler-Meßkopf, bestehend aus einem Ultraschall-Sende-Empfangs-Element (171), vorzugsweise einem Piezoelement, einem Kleber- und Filterteil (172), einem Drucksensor (173) zur Ermittlung der Anpreßkraft, einem Hilfssensorblock (174), einer Steuer- und Auswerteelektronik (175), einem Gehäuse (176), einem Anschlußstecker (177) für zusätzliche Blutdruck-Meßsensoren, sowie Referenz-Blutdrucksensoren, einem Kabel mit Stekker (178), wobei der Meßkopf (170) eine eigene Stromversorgungseinheit sowie eine Sende-Empfangseinheit mit Antenne (179) für die Übertragung von Meßdaten und Steuerdaten besitzt, wobei die Sende-Empfangseinheit (179) entweder im Simplex- und/oder Duplex-Sendemodus ar beiten kann und die Daten bei Verwendung mehrerer solcher Vorrichtungen codiert und/oder in zeitgesteuerter, gepackter Form überträgt, um die Daten gegebenenfalls an ein Hauptgerät zu übertragen, wobei dieses Gerät auch mobil sein kann und wobei die Datenübertragung, sowie die Energieversorgung über Lichtleiter erfolgen kann.

48. Vorrichtung nach einem der Ansprüche 11 bis 47, dadurch gekennzeichnet, daß der Meß-Drucksensor (1, 3, 4) bzw. der Referenz-Drucksensor (6, 6n) auf einem Meßkopf, vorzugsweise auf einem Ultraschall-Doppler-Meßkopf angebracht ist, wobei der Ultraschall-Doppler-Meßkopf, um einen weiten Frequenzbereich überdekken zu können, aus einem Array von Sende-Empfangselementen, vorzugsweise Piezoelementen, Folien, Keramik, Substraten od.dgl. aufgebaut ist.

49. Vorrichtung nach einem der Ansprüche 11 bis 48, dadurch gekennzeichnet, daß Einrichtungen zur Messung der Atmungsaktivität der Testperson in Frequenz und Amplitude vorgesehen sind.

Fig.1

Fig.2

Fig.3

Fig.4

Fig.5

Fig.6

Fig.7

Fig.8

Fig.9

Fig. 10

Fig. 11

Fig. 12

Fig. 13

Fig. 14

Fig. 15

Fig. 16

Fig. 17

Fig. 18

Europäisches
Patentamt

**EUROPÄISCHER RECHERCHENBERICHT**

Nummer der Anmeldung

EP    90 89 0003
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | WO-A-8601704 (BOMED MEDICAL MANUFACTURING LTD.) <br> * Zusammenfassung * <br><br> * Seite 5, Zeile 5 - Seite 9, Zeile 7 * <br> * Seite 13, Zeile 4 - Seite 18, Zeile 18 * <br> * Seite 26, Zeile 27 - Seite 29, Zeile 30; Figuren * <br> --- | 1-4, 6, 8-11, 13-17, 19 | A61B5/022 |
| Y,D | EP-A-256159 (NIPPON COLIN CO., LTD.) <br> * das ganze Dokument * | 1-4, 6, 8, 11, 13-17, 19 | |
| A | | 9, 25, 28, 45 <br> --- | |
| Y | MEDICAL AND BIOLOGICAL ENGINEERING AND COMPUTING. <br> vol. 21, no. 4, Juli 1983, STEVENAGE GB <br> Seiten 424 - 429; Chai Shang-da et al.: "Noninvasive determination of arterial compliance" <br> * das ganze Dokument * | 1-4, 6, 8, 11, 13-17, 19 | |
| | | | **RECHERCHIERTE SACHGEBIETE (Int. Cl.5 )** |
| A | | 32 | A61B |
| A | WO-A-8503211 (JOHNSON MATTHEY PUBLIC LIMITED COMPANY) <br> * Zusammenfassung * <br><br> * Seite 2, Zeile 10 - Seite 3, Zeile 25 * <br> * Seite 4, Zeilen 19 - 27 * <br> * Seite 7, Zeile 7 - Seite 9, Zeile 20; Figuren * <br> --- | 1-4, 6_8-11, 18, 19, 45-48 | |
| A | IEEE TRANSACTIONS ON BIO-MEDICAL ENGINEERING. <br> vol. 18, no. 1, Januar 1971, NEW YORK US <br> Seiten 56 - 59; j.weinman et al.: "The shift of arterial pulse-wave foot on recordings with a rising or falling baseline" <br> * das ganze Dokument * | 1, 2, 8, 9 | |

**Der vorliegende Recherchenbericht wurde für alle Patentansprüche erstellt**

| Recherchenort | Abschlußdatum der Recherche | Prüfer |
|---|---|---|
| DEN HAAG | 28 MAERZ 1990 | FERRIGNO A. |

Europäisches
Patentamt

EUROPÄISCHER RECHERCHENBERICHT

Nummer der Anmeldung

EP    90 89 0003
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | MEDICAL AND BIOLOGICAL ENGINEERING AND COMPUTING. vol. 24, no. 3, Mai 1986, STEVENAGE GB Seiten 255 - 260; Masahiko Okada et al.: "Estimation of arterial pulse wave velocities in the frequency domain: method and clinical considerations" * das ganze Dokument * | 1, 2, 8 | |
| A | US-A-3090377 (P.F.SALISBURY ET AL.) * das ganze Dokument * | 1-3, 11-17, 32-35, 41 | |
| A | US-A-2875750 (H. BOUCKE ET AL.) * das ganze Dokument * | 1-3, 6, 8, 10-17, 41 | |
| A | US-A-4321929 (LEMELSON ET AL.) * das ganze Dokument * | 30 | RECHERCHIERTE SACHGEBIETE (Int. Cl.5 ) |
| A | US-A-4776339 (J.M.SCHREIBER) * das ganze Dokument * | 31, 36 | |
| A | FR-A-2405694 (A.H.SACKS) * Seite 6, Zeile 39 - Seite 7, Zeile 9; Figur 1 * | 36 | |
| A | GB-A-2191587 (SIGNAL TECHNOLOGY CO. LTD.) * das ganze Dokument * | 39, 44 | |
| A | JAPAN ELECTRONIC ENGINEERING vol. 24, November 1968, TOKYO JP Seiten 30 - 33; SHIGERU MIURA: "PRESENT STANDING OF PATIENT MONITORING SYSTEMS IN JAPAN" * das ganze Dokument * | 36, 40, 49 | |

Der vorliegende Recherchenbericht wurde für alle Patentansprüche erstellt

| Recherchenort | Abschlußdatum der Recherche | Prüfer |
|---|---|---|
| DEN HAAG | 28 MAERZ 1990 | FERRIGNO A. |

Europäisches
Patentamt

**EUROPÄISCHER RECHERCHENBERICHT**

Nummer der Anmeldung

EP    90 89 0003
Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-4127114 (M. BRETSCHER)<br>* das ganze Dokument *<br>----- | 46-48 | |

**RECHERCHIERTE SACHGEBIETE (Int. Cl.5 )**

Der vorliegende Recherchenbericht wurde für alle Patentansprüche erstellt

| Recherchenort | Abschlußdatum der Recherche | Prüfer |
|---|---|---|
| DEN HAAG | 28 MAERZ 1990 | FERRIGNO A. |